# EUROPEAN PATENT APPLICATION

(11) **EP 2 281 542 A1**
(43) Date of publication of application: **09.02.2011**
(21) Application number: 09166680.0
(22) Date of filing: 29.07.2009
(51) Int. Cl.: A61J 1/06, A61J 1/14

(54) **Cartridge for drugs**

(71) Applicant: F. Hoffmann-La Roche AG, 4070 Basel (CH)
(72) Inventor: Büttiker, Jean-Pierre, 4108 Witterswil (CH)
(74) Representative: Bohest AG

(57) **Abstract**

A cartridge (11) for a drug comprises a barrel (12) with a chamber (19) defined by a tubular wall (15) of the barrel (12), sealing means (25, 27) associated with the distal end (13) of the barrel (12) and a piston (21) associated with the open proximal end (14) of the tubular barrel (12). The barrel (12) has a proximal end portion (41), a distal end portion (43) and a shoulder portion (42) which extends between the proximal end portion (41) and the distal end portion (43) of the barrel (12). The piston (21) has a proximal end portion (44), a distal end portion (46), and a shoulder portion (45) which extends between the proximal end portion (44) and the distal end portion (46) of the piston (21). The shoulder portion (45) of the piston (21) has an outer surface which contacts the inner surface of the shoulder portion of the barrel (12) when the piston (21) is displaced as far as possible towards the distal end (13) of the barrel (12).

## Description

The invention concerns a cartridge for drugs, in particular a disposable, pre-fillable cartridge for drugs.

A drawback of known cartridges for drugs is that a not insubstantial undeliverable dead volume of a liquid medicament remains in the cartridge after delivery of the medicament contained in the cartridge. This drawback is generally disadvantageous, but is particularly disadvantageous in the case of expensive medicaments.

An aim of the invention is therefore to provide a cartridge suitable for drugs and having a structure that makes it possible to at least greatly reduce such dead volume.

According to the invention the above aim is achieved by means of a cartridge as it is defined by the features of the independent claim. Embodiments of the drug cartridge according to the invention are the subject of the dependent claims.

The main advantage obtained with a drug cartridge according to the invention is that the dead volume that remains in the cartridge after delivery of a liquid medicament contained in the cartridge is greatly reduced, and that this is achieved at low cost.

The invention will now be described with the aid of preferred embodiments and with reference to the accompanying drawings. These embodiments are set forth to improve the understanding of the invention, but are not to be construed as limiting. In the drawings:
- Fig. 1: shows a schematic cross-sectional view of a first embodiment of a cartridge according to the invention,
- Fig. 2: shows a schematic cross-sectional view of a cartridge having the structure of the one shown in Fig. 1, but comprising a barrel having a length greater than that shown in Fig. 1,
- Fig. 3: shows a schematic cross-sectional view of a cartridge again having the structure of the one shown in Fig. 1, but comprising a barrel having a length still greater than that shown in Fig. 2,
- Fig. 4: is an enlarged partial cross-sectional view of the cartridge shown in Fig. 1, with the piston in its end position,
- Fig. 5: is also an enlarged partial cross-sectional view of the cartridge shown in Fig. 1, but additionally showing a hollow needle inserted through the septum 25.
- Fig. 6: shows a schematic cross-sectional view of a second embodiment of a cartridge according to the invention,
- Fig. 7: shows a schematic cross-sectional view of a cartridge having the structure of the one shown in Fig. 6, but comprising a barrel having a length greater than that shown in Fig. 6,
- Fig. 8: shows a schematic cross-sectional view of a cartridge having also the structure of the one shown in Fig. 6, but comprising a barrel having a length still greater than that shown in Fig. 7.
- Fig. 9: is an enlarged partial cross-sectional view of the drug cartridge shown in Fig. 6,
- Fig. 10: is another enlarged partial cross-sectional view of the cartridge shown in Fig. 6, but additionally showing a hollow needle inserted through the stopper, and
- Fig. 11: is an enlarged partial crosssectional view of a further embodiment of the cartridge according to the invention.

Embodiments are described hereinafter with reference to the accompanying drawings.In this disclosure, a convention is followed wherein the distal end of a component of the drug cartridge is the end closest to a patient and the proximal end of the component is the end remote from the patient and closest to a practitioner when the latter uses the cartridge to inject a medicament into the patient.

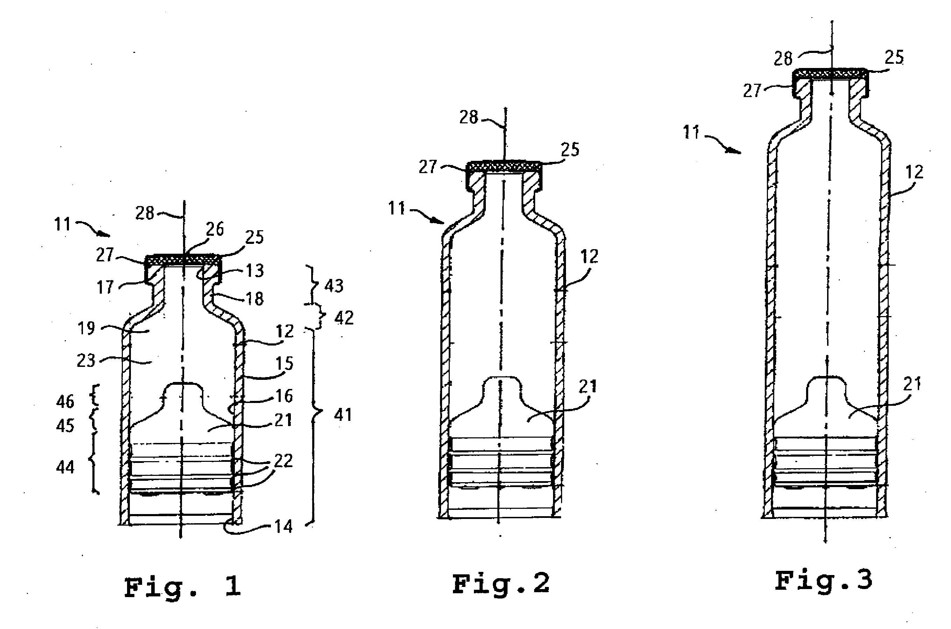

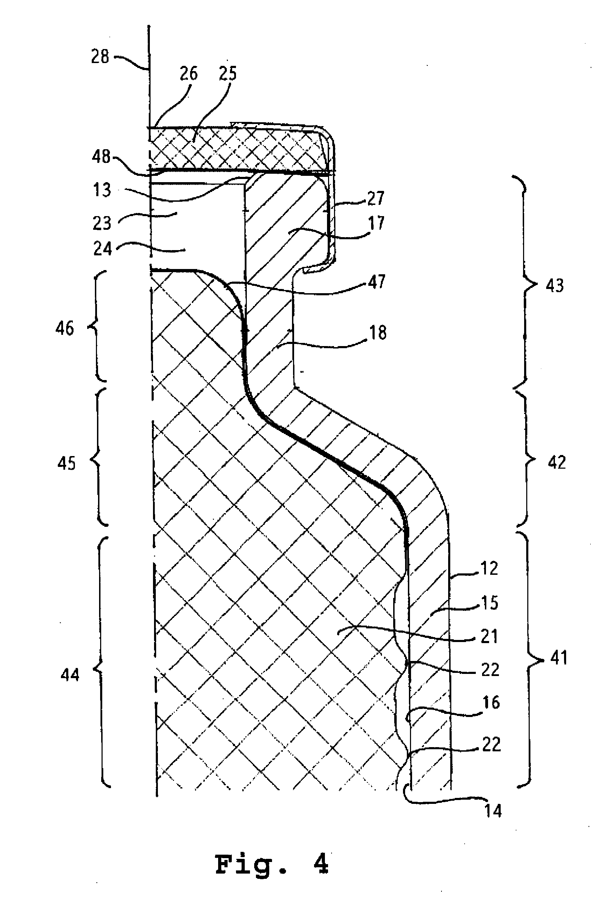

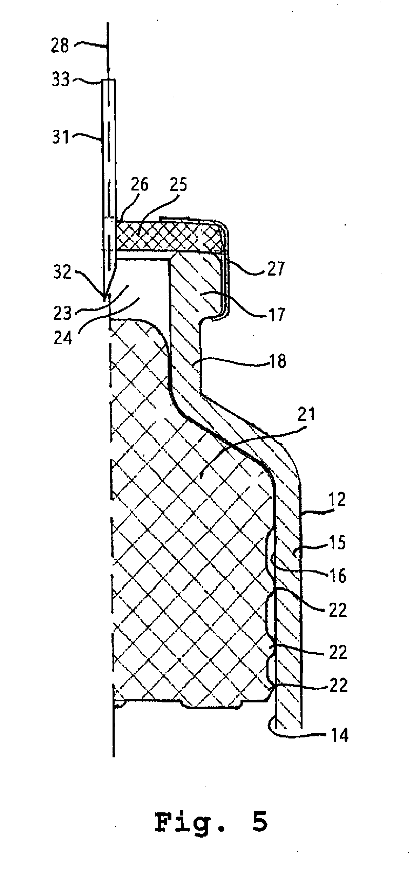

**Figures 1 to 5** show a first embodiment of a drug cartridge 11 according to the invention. Each of the cross-sectional views of Figures 4 and 5 show one half of the cartridge, the other half is a mirror image of that half about the symmetry axis 28.

As can be appreciated in particular from the enlarged views shown in Figures 4 and 5, cartridge 11 comprises a generally tubular barrel 12, sealing means comprising a piercable septum 25 and a sealing cap 27, and a piston 21.

Barrel 12 is preferably a glass barrel made of a glass which does not physically or chemically interact with a liquid medicament contained in the barrel in any manner to alter the strength, quality, or purity beyond the official requirements under the ordinary or customary conditions of handling, shipment, storage, sale and use. By way of example only, a suitable glass type for barrel 12 is glass commercially available under the name FIOLAX®.

Siliconizing of the inner surface of glass barrel 12 is carried out with a standard inline-washing-spraying process followed by a bonding of silicon oil in a burning-in tunnel. The main purpose of the siliconizing is to reduce friction between the glass barrel 12 and the piston 21 to facilitate movement of piston 21 along tubular wall 15 of glass barrel 12 when being actuated, e.g. with the aid of a plunger rod. A further advantageous aspect of the siliconizing is to prevent undesired absorption of active ingredients of a liquid medicament by the glass walls of the barrel 12.

Barrel 12 comprises an open distal end 13, an open proximal end 14, and a chamber 19 defined by a tubular wall 15 of the barrel 12. In the embodiment shown in Fig. 1, barrel 12 has a length of 50 mm (millimeters), and cartridge 11 is adapted to contain 5 ml (milliliters) of a liquid medicament.

Barrel 12 has a proximal end portion 41, a distal end portion 43 and a shoulder portion 42. The proximal end portion 41 of barrel 12 has an inner diameter of e.g. 19 mm and an outer diameter of e.g. 22 mm. The distal end portion 43 of barrel 12 comprises a rim portion 17 surrounding the opening at the distal end 13 of barrel 12 and a neck 18 having a diameter that is smaller than the diameter of barrel 12. Neck 18 has an inner diameter of e.g. 7 mm and an outer diameter of e.g. 10.5 mm. The shoulder portion 42 of barrel 12 extends between the proximal end portion 41 and the distal end portion 43 of the barrel 12, and tapers conically from the proximal end portion 41 to the distal end portion.

The sealing means comprising septum 25 and sealing cap 27 are connected to the distal end 13 of the tubular barrel 12 for sealing the distal end 13 of the tubular barrel 12. Septum 25 is placed upon the rim portion 17 of barrel 12 and is secured thereto by sealing cap 27. The sealing cap 27 is e.g. a standard aluminum sealing ring, which has been crimped so as to secure septum 25 to rim portion 17 of barrel 12.

As shown in particular by Figures 4 and 5, septum 25 is disk-shaped and the inner surface of septum 25 is preferably coated with a film 48 of a Teflon®-like material (polytetrafluoroethylene). This coating prevents undesirable interactions between a liquid medicament stored in chamber 19 of cartridge 11 and the material of septum 25.

Septum 25 is preferably made of a suitable rubber material, e.g. of Daikyo D777 rubber.

Septum 25 has at least a portion 26 which is piercable by a hollow needle 31 having a sharpened end at its proximal end tip 32 as shown in Fig. 5.

Hollow needle 31 has a distal end tip 33 which may have the shape shown in Fig. 5 or which may be also a sharpened end. Hollow needle 31 is used for transferring the liquid medicament contained in cartridge 11 to another container, e.g. to the chamber of a syringe, or for injecting the liquid medicament into a patient. In this case, a hollow needle 31 having a sharpened end at both its proximal and its distal end tips is used.

Piston 21 - in its starting position - is arranged at the open proximal end 14 of the tubular barrel 12 in sliding fluid-tight engagement with the tubular wall 15 of the barrel 12 for selective engagement with an advancing member (not shown in the drawings) so that distally directed forces on the piston 21 eject a liquid medicament 23 pre-filled into the chamber 19 from the cartridge 11 through a hollow needle inserted through septum 25 as shown in Fig. 5. Piston 21 is slidably arranged at the proximal end portion 41 of barrel 12. Piston 21 is axially movable for ejecting a liquid medicament contained in chamber 19 of cartridge 11, but is axially stationary during normal storage and handling of cartridge 11. Distally directed forces can be applied to the piston 21 e.g. by a plunger rod (not shown in the drawings) or other functionally similar structure brought into contact with the proximal end of piston 21 and used as an advancing member.

Piston 21 has a proximal end portion 44, a distal end portion 46 and a shoulder portion 45. The proximal end portion 44 of piston 21 has a diameter which is such that piston 21 frictionally and sealingly engages the inner surface 16 of the tubular wall 15 of the proximal end portion 41 of the tubular barrel 12. The proximal end portion 44 of piston 21 has circumferential ribs 22.

Figures 4 and 5 show piston 21 in its end position after it has been displaced as far as possible towards the distal end 13 of the barrel 12.

As shown in Figures 4 and 5, the distal end portion 46 of piston 21 has a diameter which is such that the distal end portion 46 of the piston 21 contacts the inner surface of the neck 18 of the barrel 12 when the piston 21 is displaced as far as possible towards the distal end 13 of the barrel 12. The shoulder portion 45 of piston 21 extends between the proximal end portion 44 and the distal end portion 46 of piston 21 and tapers conically from the proximal end portion 44 to the distal end portion 46. The shoulder portion 45 of piston 21 has an outer surface which contacts the inner surface of the shoulder portion of the barrel 12 when the piston 21 is displaced as far as possible towards the distal end 13 of the barrel 12.

As shown in Figures 4 and 5, the shape of the outer surfaces of the shoulder portion 45 and of the distal end portion 46 of the piston 21 preferably closely match the shape of the inner surfaces of the shoulder portion 42 and of the distal end portion 43 of the barrel 12.

As also shown in Figures 4 and 5, the outer surfaces of the shoulder portion 45 and of the distal end portion 46 of the piston 21 as well as the inner surfaces of the shoulder portion 42 and of the distal end portion 43 of the barrel 12 preferably comprise no sharp edge.

The above described features of the shape of piston 21 and of the shoulder and distal end portions of the barrel 12 contribute substantially to the great reduction of the dead volume 24 of liquid medicament which remains in the barrel after delivery thereof through hollow needle 31 by moving piston 21 as far as possible towards the distal end 13 of the barrel 12 until piston 21 reaches its end position shown in Figures 4 and 5.

As shown in Figures 4 and 5, the outer surfaces of the shoulder portion 45 and of the distal end portion 46 of the piston 21 are preferably coated with a film 47 of a Teflon®-like material. This coating prevents undesirable interactions between a liquid medicament stored in chamber 19 of cartridge 11 and the material of piston 21.

Piston 21 is preferably made of a suitable rubber material, e.g. of Daikyo D777 rubber.

In one embodiment, the above described cartridge 11 is a disposable, pre-fillable cartridge the chamber 19 of which can be filled with a liquid medicament 23. The liquid medicament can be retained in chamber 19 by the sealing means 25, 27, and the piston 21, so that distally directed forces on the piston 21 eject the liquid medicament 23 from the cartridge 11 through a hollow needle 31 which can be inserted through the septum 25.

The embodiments of the cartridge 11 shown in Figures 2 and 3 have in principle the same structure and dimensions as the above described embodiment of the cartridge 11 shown in Fig. 1 and differ therefrom only in their respective lengths of the barrel 12. In the embodiment shown in Fig. 2, barrel 12 has a length of 66.5 mm (millimeters), and cartridge 11 is adapted to contain 10 ml (milliliters) of a liquid medicament. In the embodiment shown in Fig. 3, barrel 12 has a length of 84.3 mm, and drug cartridge 11 is adapted to contain 15 ml of a liquid medicament. Additional standard volumes of such cartridges are 1.7 ml (milliliters) and 3 ml (milliliters), respectively, and the dimensions of the cartridges or the various parts thereof can be adapted accordingly.

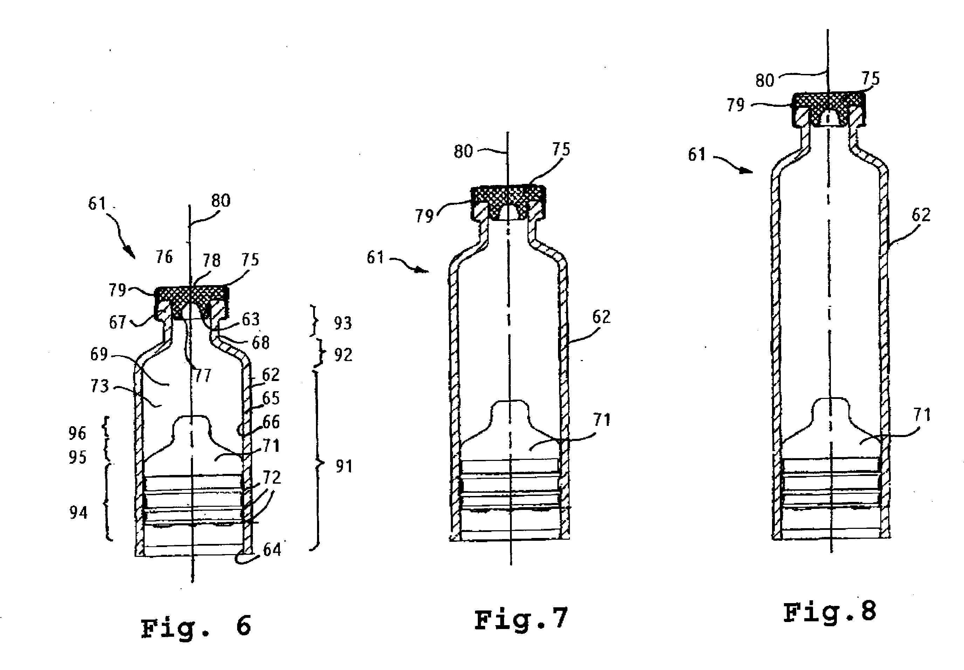

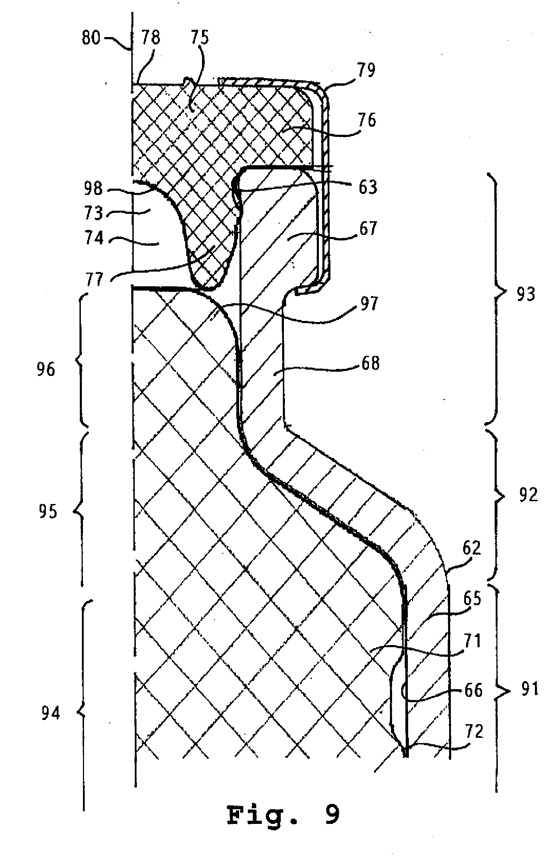

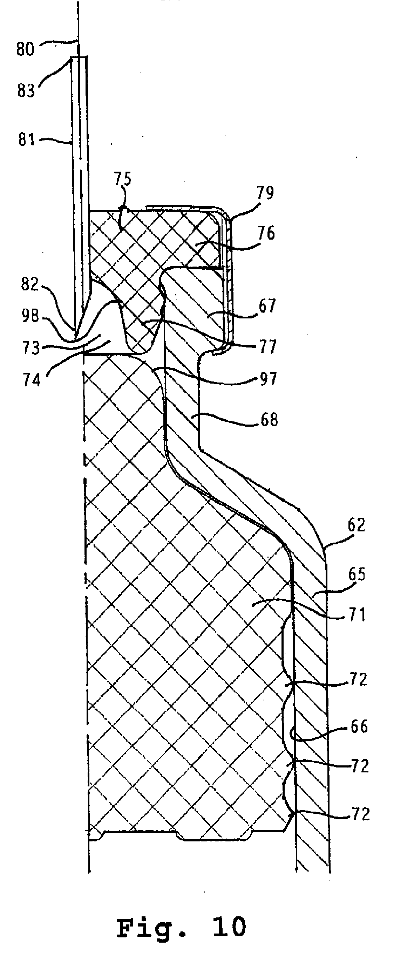

**Figures 6 to 10** show a second embodiment of a cartridge 61 according to the invention. Each of the cross-sectional views of Figures 9 and 10 shows one half of the cartridge, the other half is a mirror image of that half about the symmetry axis 80.

As can be appreciated in particular from the enlarged views shown in Figures 9 and 10, cartridge 61 comprises a generally tubular barrel 62, sealing means comprising a piercable stopper 75 and a sealing cap 79, and a piston 71.

Barrel 62 is preferably a glass barrel made of a glass which does not physically or chemically interact with a liquid medicament contained in the barrel in any manner to alter the strength, quality, or purity beyond the official requirements under the ordinary or customary conditions of handling, shipment, storage, sale and use. By way of example only, a suitable glass type for barrel 62 is glass commercially available under the name FIOLAX®.

Siliconizing of the inner surface of glass barrel 62 is carried out with a standard inline-washing-spraying process followed by a bonding of silicon oil in a burning-in tunnel. The main purpose of the siliconizing is to reduce friction between the glass barrel 62 and the piston 71 so that the piston 71 can be easily moved along the tubular wall 65 of the glass barrel 62. A further advantageous aspect of the siliconizing is to prevent undesired absorption of active ingredients of a liquid medicament by the glass walls of the barrel 62.

Barrel 62 comprises an open distal end 63, an open proximal end 64, and a chamber 69 defined by a tubular wall 65 of the barrel 62. In the embodiment shown in Fig. 6, barrel 62 has a length of 50 mm (millimeters), and cartridge 61 is adapted to contain 5 ml (milliliters) of a liquid medicament.

Barrel 62 has a proximal end portion 91, a distal end portion 93 and a shoulder portion 92. The proximal end portion 91 of barrel 62 has an inner diameter of e.g. 69 mm and an outer diameter of e.g. 72 mm. The distal end portion 93 of barrel 62 comprises a rim portion 67 surrounding the opening at the distal end 63 of barrel 62 and a neck 68 having a diameter that is smaller than the diameter of barrel 62. Neck 68 has an inner diameter of e.g. 7 mm and an outer diameter of e.g. 10.5 mm. The shoulder portion 92 of barrel 62 extends between the proximal end portion 91 and the distal end portion 93 of the barrel 62 and tapers conically from the proximal end portion 91 to the distal end portion.

The sealing means comprising stopper 75 and a sealing cap 79 are connected to the distal end 63 of the tubular barrel 62 for sealing the distal end 63 of the tubular barrel 62. Stopper 75 is secured to the rim portion 67 of barrel 62 by sealing cap 79. The sealing cap 79 is e.g. a standard aluminum sealing ring which has been crimped so as to secure stopper 75 to the rim portion 67 of barrel 62.

Stopper 75 has a planar rim portion 76 placed upon the rim portion 67 of the barrel 62 and a tubular portion 77 having a diameter slightly greater than the internal diameter of the opening at the distal end 63 of the barrel 62 to provide a tight seal.

As shown in particular by Figures 9 and 10, the inner surface of stopper 75 is preferably coated with a film 98 of a Teflon®-like material. This coating prevents undesirable interactions between a liquid medicament stored in chamber 69 of cartridge 61 and the material of stopper 75.

One example of an appropriate stopper 75 is a Daikyo D777 rubber stopper.

The planar rim portion 76 of stopper 75 has at least a portion 78 which is piercable by a hollow needle 81 having a sharpened end at its proximal end tip 82 as shown in Fig. 10.

Hollow needle 81 has a distal end tip 83 which may have the shape shown in Fig. 10 or which may be also a sharpened end. Hollow needle 81 is used for transferring the liquid medicament contained in cartridge 61 to another container, e.g. to the chamber of a syringe, or for injecting the liquid medicament into a patient. In this case, a hollow needle 81 having a sharpened end at both its proximal and its distal end tips is used.

Piston 71 - in its starting position - is arranged at the open proximal end 64 of the tubular barrel 62 in sliding fluid-tight engagement with the tubular wall 65 of the barrel 62 for selective engagement with an advancing member (not shown in the drawings) so that distally directed forces on the piston 71 eject a liquid medicament 73 pre-filled into chamber 69 from the cartridge 61 through a hollow needle inserted through stopper 75 as shown in Fig. 10. Piston 71 is slidably disposed in the proximal end portion 91 of barrel 12. Piston 71 is axially movable for ejecting a liquid medicament contained in chamber 69 of cartridge 61, but is axially stationary during normal storage and handling of cartridge 61. Distally directed forces can be applied to the piston 71 e.g. by a plunger rod (not shown in the drawings) or other functionally similar structure brought into contact with the proximal end of piston 71 and used as advancing member.

Piston 71 has a proximal end portion 94, a distal end portion 96 and a shoulder portion 95. The proximal end portion 94 of piston 71 has a diameter which is such that piston 71 frictionally and sealingly engages the inner surface 66 of the tubular wall 65 of the proximal end portion 91 of the tubular barrel 62. The proximal end portion 94 of piston 71 has circumferential ribs 72.

Figures 9 and 10 show piston 71 in its end position after it has been displaced as far as possible towards the distal end 63 of the barrel 62.

As shown in Figures 9 and 10, the distal end portion 96 of piston 71 has a diameter which is such that the distal end portion 96 of the piston 71 contacts the inner surface of the neck 68 of the barrel 62 when the piston 71 is displaced as far as possible towards the distal end 63 of the barrel 62. The shoulder portion 95 of piston 71 extends between the proximal end portion 94 and the distal end portion 96 of piston 71 and tapers conically from the proximal end portion 94 to the distal end portion 96. The shoulder portion 95 of piston 71 has an outer surface which contacts the inner surface of the shoulder portion of the barrel 62 when the piston 71 is displaced as far as possible towards the distal end 63 of the barrel 62.

As shown in Figures 9 and 10, the shape of the outer surfaces of the shoulder portion 95 and of the distal end portion 96 of the piston 71 closely match the shape of the inner surfaces of the shoulder portion 92 and of the distal end portion 93 of the barrel 62.

As also shown in Figures 9 and 10, the outer surfaces of the shoulder portion 95 and of the distal end portion 96 of the piston 71 as well as the inner surfaces of the shoulder portion 92 and of the distal end portion 93 of the barrel 62 preferably comprise no sharp edge.

The above described features of the shape of piston 71 and of the shoulder and distal end portions of the barrel 62 contribute substantially to the great reduction of the dead volume 74 of liquid medicament which remains in the barrel 62 after delivery thereof through hollow needle 81 by moving piston 71 as far as possible towards the distal end 13 of the barrel 62 until piston 71 reaches its end position shown in Figures 9 and 10.

As shown in Figures 9 and 10, the outer surfaces of the shoulder portion 95 and of the distal end portion 96 of the piston 71 are preferably coated with a film 97 of a Teflon®-like material. This coating prevents undesirable interactions between a liquid medicament stored in chamber 69 of cartridge 61 and the material of piston 71.

Piston 71 is preferably made of a suitable rubber material, e.g. of Daikyo D777 rubber.

In a preferred embodiment, the above described cartridge 61 is a disposable, pre-fillable cartridge the chamber 69 of which can be filled with a liquid medicament 73. The liquid medicament can be retained in chamber 69 by the sealing means 75, 79 and the piston 71, so that distally directed forces on the piston 71 eject the medicament 73 from the cartridge 61 through a hollow needle 81 inserted through the stopper 75.

The embodiments of the cartridge 61 shown in Figures 7 and 8 have in principle the same structure and dimensions as the above described embodiment of the cartridge 61 shown in Fig. 6 and differ therefrom only in their respective lengths of the barrel 62. In the embodiment shown in Fig. 7, barrel 62 has a length of 66.5 mm (millimeters), and drug cartridge 61 is adapted to contain 10 ml (milliliters) of a liquid medicament. In the embodiment shown in Fig. 8, barrel 62 has a length of 84.3 mm, and drug cartridge 61 is adapted to contain 15 ml of a liquid medicament.

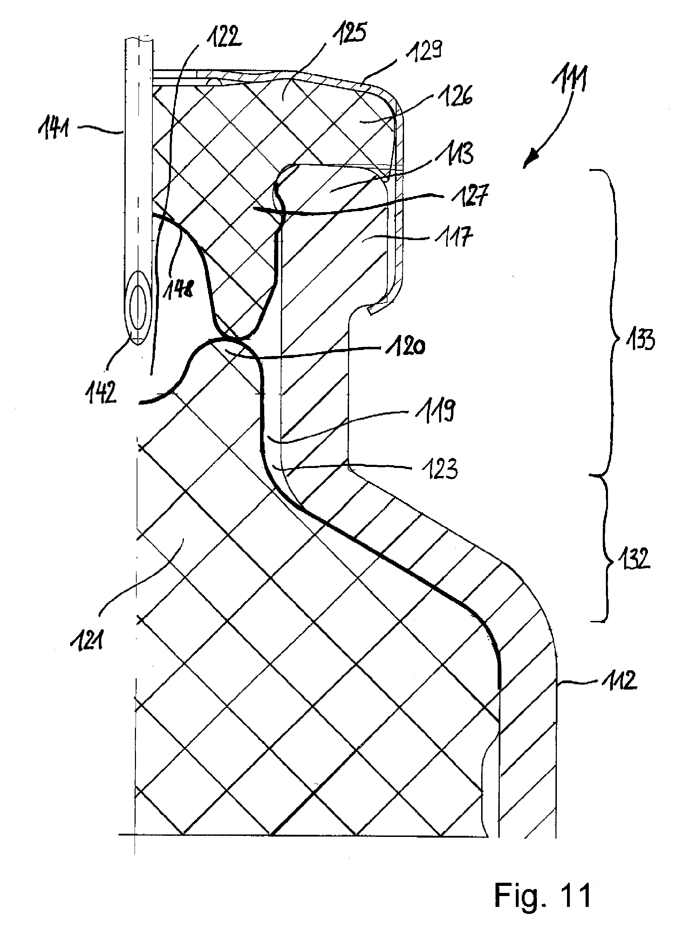

Another embodiment of the cartridge according to the invention is shown in **Fig. 11** in an enlarged partial cross-sectional view. Cartridge 111 comprises a generally tubular barrel 112 defining a chamber 119 wich can be filled with a liquid medicament 123. Barrel 112 has a proximal end portion 131, a distal end portion 133 and a shoulder portion 132. The liquid medicament 123 can be retained in chamber 119 by sealing means which comprise a piercable stopper 125 and a sealing cap 129 on one hand, and which comprise piston 121 on the other hand.

As already mentioned for the other embodiments, barrel 112 is preferably a glass barrel made of a glass which does not physically or chemically interact with the liquid medicament contained in the barrel in any manner to alter the strength, quality or purity beyond the official requirements under the ordinary or customary conditions of handling, shipment, storage, sale and use. By way of example only, a suitable glass type for barrel 112 is glass commercially available under the name FIOLAX®.

The sealing means comprising stopper 125 and sealing cap 129 are connected to the distal end portion 133 of tubular barrel 112 for sealing the distal end of tubular barrel 112. Stopper 125 is secured to a rim portion 117 of barrel 112 by sealing cap 129. Sealing cap 129 is e.g. a standard aluminum sealing cap which has been crimped so as to secure stopper 125 to rim portion 117 of barrel 112.

Stopper 125 has a planar rim portion 126 placed upon rim portion 117 of barrel 112, and a tubular portion 127 having a diameter slightly greater than the internal diameter of the opening at the distal end 113 of barrel 112. The inner surface of stopper 125 is preferably coated with a film 148 of a Teflon®-like material. This coating prevents undesirable interactions between a liquid medicament stored in chamber 119 of cartridge 111 and the material of stopper 125.

One example of an appropriate stopper 125 is a Daikyo D777 rubber stopper.

The planar rim portion 126 of stopper 125 has at least a portion 128 which is piercable by a hollow needle 141 having a sharpened end at its proximal end tip 142 as shown in Fig. 11. Hollow needle 141 is used for transferring the liquid medicament contained in cartridge 111 to another container, e.g. to the chamber of a syringe, or is used for injecting the liquid medicament into a patient.

Piston 121 has a recess 122 for accommodating the needle tip which is advantageous inasmuch as once piston 121 reaches its foremost position shown in Fig. 11, it does not abut against the proximal end tip 142 of needle 141 but rather protrusions 120 of piston 121 abut against the tips of the tubular portion 127 of stopper 125. Thus, the proximal end tip 142 of needle 141 does not come into contact with stopper 125 at any time.

In this embodiment it is possible that the needle is a part which is fixed in the end face of a tubular needle holder component (not represented in the drawings) which can simply be moved over the distal end 113 of barrel 112 until the needle holder component is snap-fitted onto the said distal end 113 of barrel 112. Upon moving the needle holder component onto the distal end 113 of barrel 112 stopper 125 is penetrated by the needle and once the snap of the needle holder component occurs, the needle reaches into the needle space defined by the recesses of the stopper 125 and the piston 121 when the piston 121 is in its end position shown in Fig. 11.

Another embodiment of the cartridge according to the invention which is not represented in the drawings relates to a dual chamber cartridge, with one chamber containing a lyophilized product and the other chamber containing the buffer, in which the lyophilized product is solved immediately prior to administration. Such an embodiment of the cartridge may comprise a similar piston, septum or stopper as described in the embodiments shown in the drawings. However, means must be provided in addition which first allow the buffer contained in one chamber to enter the other chamber containing the lyophilized product and to allow the lyophilized product to solve in the buffer, and then the cartridge may work in the manner described with reference to the drawings. Such additional means are well-known in the art and may comprise, by way of example, a througbore in the piston connecting the two chambers and a membrane arranged therebetween. Upon exerting pressure on the buffer contained in one chamber, e.g. with the aid of a suitable plunger rod, the membrane in the piston ruptures so that the buffer flows through the throughbore in the piston and enters the other chamber containing the lypohilized product. Further movement of the plunger rod towards the piston causes the remaining buffer to also enter the chamber containing the lyophilized product. Upon completion of the solving process, the medicament can then be administered.

Although specific embodiments of the invention have been described using specific terms, such description is for illustrative purposes only, and it is to be understood that changes and variations obvious to the skilled artisan are to be considered within the scope of the invention, which is defined by the appended claims.

## Claims

1. A cartridge (11, 61, 111) for drugs comprising:
- a generally tubular barrel (12, 62, 112) having an open distal end (13, 63) and an open proximal end (14, 64), with a chamber (19, 69, 119) defined by a tubular wall (15, 65) of the barrel (12, 62),
said barrel (12, 62, 112) having a proximal end portion (41, 91) having a first inner diameter, a distal end portion (43, 93, 133) having a second inner diameter that is smaller than said first diameter, and a shoulder portion (42, 92) extending between the proximal end portion (41, 91) and the distal end portion (43, 93, 133) of the barrel (12, 62, 112), the inner surface of the shoulder portion (42,92) tapering from the first inner diameter of the proximal end portion (41,91) to the second inner diameter of of the distal end portion (43, 93, 113);
- a piston (21, 71, 121) displaceably arranged in the tubular barrel (12, 62, 112) in sliding fluid-tight engagement with the tubular wall (15, 65) of the barrel (12, 62, 112),
said piston (21, 71, 121) having a proximal end portion (44, 94) having a first outer diameter corresponding to the first inner diameter of the proximal portion (41, 91) of the barrel (12, 62, 112), so that the piston (21, 71, 121) frictionally and sealingly engages the inner surface (16, 66) of the tubular wall (15, 65) of the proximal end portion (41, 91) of the tubular barrel (12, 62, 112), a distal end portion (46, 96) having a second outer diameter corresponding to the second inner diameter of the distal end portion (43, 93, 133) of the barrel (12, 62, 112), and a shoulder portion (45, 95) extending between the proximal end portion (44, 94) and the distal end portion (46, 96) of the piston (21, 71, 121), said shoulder portion (45, 95) of the piston (21, 71, 121) having an outer surface which is shaped such that it corresponds to the inner surface of the shoulder portion (42, 92) of the barrel (12, 62), and
- sealing means (25, 27, 75, 79, 125, 129) connected to the distal end (13, 63) of the tubular barrel (12, 62, 112) for sealing the distal end (13, 63) of the tubular barrel (12, 62, 112).

2. A cartridge according to claim 1, wherein the sealing means comprise a disk-shaped septum (25) placed upon the distal end portion (43) of the barrel (12) and a sealing cap (27) for securing the septum (25) to the distal end portion (43) of the barrel (12).

3. A cartridge according to claim 2, wherein the inner surface of the septum (25) is coated with a film (48) of a Teflon®-like material.

4. A cartridge according to claim 1, wherein the sealing means comprise a stopper (75, 125) plugged into the distal end portion (93, 133) of the barrel (62, 112) and a sealing cap (79, 129) for securing the stopper to the distal end portion (93, 133) of the barrel (62, 112).

5. A cartridge according to claim 4, wherein the inner surface of the stopper (75, 125) is coated with a film (98, 148) of a Teflon®-like material.

6. A cartridge according to any of the preceding claims, wherein the shoulder portion (42, 92) of the barrel (12, 62, 112) conically tapers from the proximal end portion (41, 91) to the distal end portion (43, 93, 133), and wherein the outer surface of the shoulder portion (45, 95) of the piston (21, 71, 121) conically tapers from the proximal end portion (44, 94) to the distal end portion (46, 96), and wherein further the shape of the outer surfaces of the shoulder portion (45, 95) and of the distal end portion (46, 96) of the piston (21, 71, 121) closely match the shape of the inner surfaces of the shoulder portion (42, 92) and of the distal end portion (43, 93, 133) of the barrel (12, 62, 112).

7. A cartridge according to any one of the preceding claims, wherein the outer surfaces of the shoulder portion (45, 95) and of the distal end portion (46, 96) of the piston (21, 71, 121) as well as the inner surfaces of the shoulder portion (42, 92) and of the distal end portion (43, 93, 133) of the barrel (12, 62, 112) comprise no sharp edge.

8. A cartridge according to any of the preceding claims, wherein the outer surfaces of the shoulder portion (45, 95) and of the distal end portion (46, 96) of the piston (21, 71, 121) are coated with a film (47, 97) of a Teflon®-like material.

9. A cartridge according to any of the preceding claims, which is designed as a disposable, pre-fillable drug cartridge (11, 61, 111) containing a liquid medicament (23, 73, 123) in the chamber (19, 69, 119) of the barrel (12, 62, 112).
